# EUROPEAN PATENT SPECIFICATION

(11) **EP 4 426 244 B1**
(45) Date of publication and mention of the grant of the patent: **11.02.2026**
(21) Application number: 22803050.8
(22) Date of filing: 04.11.2022
(51) Int. Cl.: A61F 5/44

(54) **A VALVE FOR A UROSTOMY APPLIANCE**
VENTIL FÜR EINE UROSTOMIEVORRICHTUNG
VALVE POUR APPAREIL D'UROSTOMIE

(30) Priority: 05.11.2021 GB 202115967
(43) Date of publication of application: 11.09.2024
(73) Proprietor: Salts Healthcare Limited, West Midlands B7 4AA (GB)
(72) Inventor: SMITH, Lee, Birmingham, West Midlands B7 4AA (GB); WILLIAMS, Kieran, Birmingham, West Midlands B7 4AA (GB); HEYNES, Mark, Beaconside, Staffordshire ST18 0AR (GB); SAMPEY, Phil, Beaconside, Staffordshire ST18 0AR (GB)
(74) Representative: Forresters IP LLP
(86) International application number: PCT/GB2022/052791
(87) International publication number: WO 2023/079300

(56) References cited:
- DE-A1- 102014 104 737
- US-A1- 2012 286 187

## Description

### Introduction

Embodiments of the invention relate to a valve for a urostomy valve or a urostomy or ileostomy appliance.

Urostomy appliances are well known in the field. They are typically attached to a user via an adhesive member that extends around the user's stoma with adhesive and provide a collecting volume to collect waste (mostly liquid waste) exiting the stoma. A mechanism for draining the collecting volume is often provided - typically, these are in the form of a tap, so that a user can empty waste that is collected in the collecting volume without having to remove and dispose of the entire appliance.

DE10 2014104737 discloses an ostomy bag including a valve which has a valve bushing which forms an outlet opening from a bag cavity and which has a valve plug with which the outlet opening can be closed.

According to a first aspect of the invention we provide a valve for a urostomy appliance according to the subject-matter of claim 1.

According to a second aspect of the invention we provide a urostomy or ileostomy appliance according to the subject-matter of claim 14.

Further optional features relating to all the aspects are recited in the appended claims.

In order that the present disclosure may be more readily understood, preferable embodiments thereof will now be described, by way of example only, with reference to the accompanying drawings, in which:
FIGURE 1 is a view of a urostomy appliance;
FIGURE 2 is a perspective partial view of a urostomy appliance in accordance with an embodiment of the disclosure;
FIGURE 3 is another perspective partial view of a urostomy appliance;
FIGURE 4 is another perspective partial view of a urostomy appliance;
FIGURE 5 is another perspective partial view of a urostomy appliance;
FIGURE 6 is a perspective partial view of a urostomy appliance and a connected drainage tube;
FIGURE 7 is a perspective view of a valve for a urostomy appliance;
FIGURE 8 is a partial view of a urostomy appliance in accordance with an embodiment of the disclosure;
FIGURE 9 is a perspective view of a valve for a urostomy appliance;
FIGURE 10 is another perspective partial view of a urostomy appliance; and
FIGURE 11 is a perspective partial view of a urostomy appliance and a connected drainage tube.

Referring to the figures, a urostomy appliance 10 (appliance 10) is illustrated. The urostomy appliance 10 includes a first wall 12 which has an attachment member (not shown) for attaching the appliance 10 to the user and also defines a stoma receiving opening for fitting about the stoma of the user. A second wall 14 is connected about its periphery to a periphery of the first wall 12, so that between the first and second wall 12, 14 forms a collecting volume (to receive waste from the stoma).

The urostomy appliance 10 further includes an outlet portion 20. The outlet portion 20 is formed from a first and a second outlet wall 22, 24. The first and second outlet wall 22, 24 are connected along a periphery to form a tube / conduit.

The appliance 10 also includes a valve 100 which provides an outlet 102. The valve 100 is connected to the outlet portion 20. Thus, the outlet portion 20 and the valve 100 provide a flow path between the collecting volume and the outlet 102. In other words, the outlet portion 20 forms a conduit between the collecting volume and the valve 100. Waste flowing from the collecting volume will pass through the outlet portion 20 and through the valve 100 to exit the appliance 10 through the outlet 102.

The valve 100 includes a body 110, an inlet connecting to the outlet 102 by a flow path, and a closure arrangement 120. The closure arrangement 120 is moveable between an open position and a closed position (and vice versa). In the closed position, the closure arrangement 120 blocks the outlet 102, such that liquid cannot flow through the outlet 102. In the open position, the outlet 102 is open, such that liquid is permitted to flow out of the outlet 102. It should be appreciated that waste from a urostomy is predominantly liquid. However, the valve 100 is suitable for an ileostomy - i.e. potentially higher flow rate. Further, an ileostomy will also output waste that has a more solid component and / or is or has regions of waste that are more viscous than a urostomy. It should be appreciated that the functionality of the valve 100 makes it suitable for waste that has a large component of liquid and / or is predominantly liquid.

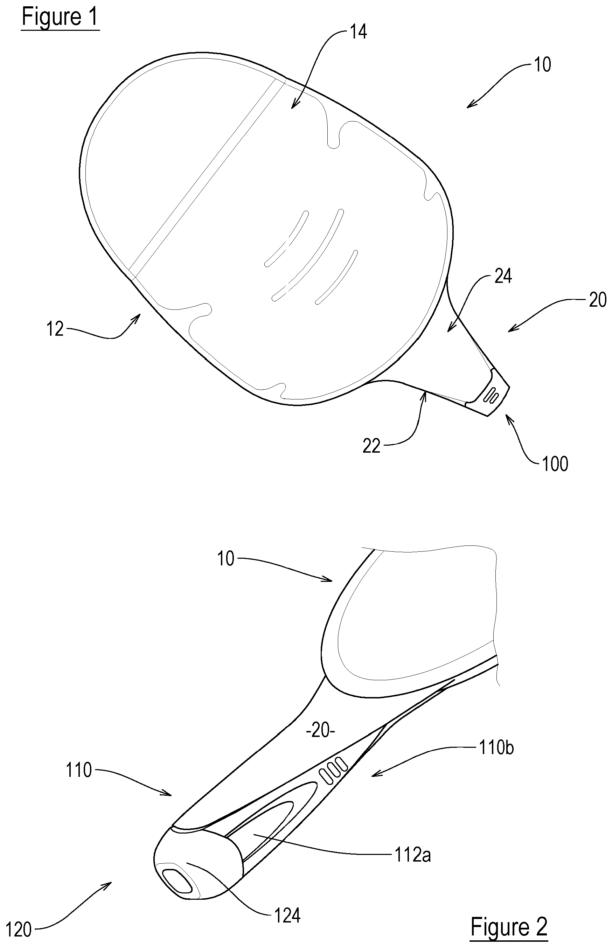

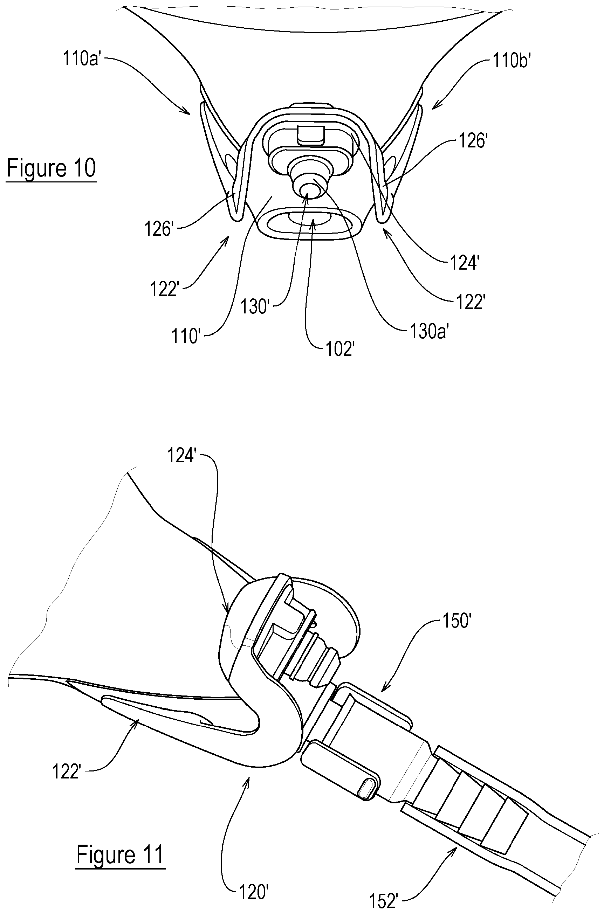

In some embodiments, one or more external surfaces of the valve 100 cooperate with one or more external surfaces of the outlet portion 20 to form a smooth transition between the outlet portion 20 and the valve 100 (see for example, figures 2 and 11). In other words, the valve 100 is incorporated into the outlet portion 20 to form a tail arrangement. Such incorporation is more comfortable for a user to wear. For example, it may reduce sharp corners and parts of the appliance 10 from digging in to a user's flesh, etc. In some embodiments, the outlet portion 20 provides the upper and lower external surfaces (of the tail arrangement) and the valve 100 provides both left and right side external surfaces. In some embodiments, the valve 100 is made of a soft and / or flexible material (e.g. to further increase the comfort of a user). More detail of how the valve is attached to an appliance is discussed below once the functionality of the valve has been discussed.

Features of the valve 100 will now be described in more detail. All embodiments discussed here may be combined with the urostomy appliance 10 structure described above.

As described above, the valve 100 includes a body 110, an inlet 104, an outlet 102 and a closure arrangement 120. The body 110 is configured to be connected to the urostomy appliance 10 (and may be attached to an outlet portion 20 as described above). The inlet and the outlet 102 are connected by a flow path (i.e. the flow path for waste out of the valve 100 and the appliance that the valve 100 is attached to). As mentioned above, the closure arrangement 120 is moveable between an open and a closed position in order to open and close the valve 100.

In some examples (such as those illustrated in figures 2 to 7), the closure arrangement 120 is detachably connectable to the body 110. In other words, the closure arrangement 120 is removable from the body 110 of the valve 100 and re-attachable as desired. Thus, the closure member 120 may be attached to and supported by the body 110 irrespective of whether the valve 100 is open or closed.

In some embodiments, the closure arrangement 120 includes a connection member 122 that provides the detachable connection to the body 110. In the illustrated example, the closure arrangement 120 includes a closure body 124 and the connection member 122.

In some embodiments, the connection member 122 includes an elongate portion 126 that extends away from the closure body 124 (and that provides the connection to the body 110). Thus, the connection member 122 provides a projection that can be attached to the body 110 of the valve 100.

The closure body 124 supports a blocking member (which blocks the outlet when in the closed position and moves to open the outlet when in the open position). In the example illustrated, the blocking member is a bung portion 130 that is configured to be positioned in the outlet 102 when the closure arrangement 120 is in its closed position (i.e. blocking waste from exiting the valve 100). Thus, the bung portion 130 is held in the outlet by a friction or push fit. The closure body 124 (and more specifically, the bung portion 130) is configured to be insertable into the outlet 102 of the valve 100 whether the connection member 122 is attached to the body 110 or not.

In some embodiments, the bung portion 130 includes a flanged part 130a that engages an internal surface inside the outlet 102. This may aid the connection between the closure and the body 110 of the valve and reduce the likelihood that the bung potion 130 will detach itself / come out of the outlet 102 accidently. In some embodiments, the bung portion 130 includes a wider or flared portion towards its end (relative to the portion that sits in the outlet 102). This permits a mechanical engagement with the valve 100 that holds the bung portion 130 in place securely.

According to the invention, the body 110 provides a first engagement formation 112 configured to receive a portion of the connection member 122. The connection member 122 also includes a corresponding engagement formation 128 that is configured to engage with the first engagement formation 112.

According to the invention,
the first engagement formation 112 is a receiving portion. In other words,
the body 110 includes a recess or opening that "receives" a portion of the connection member 122 to hold and support the closure body relative to the body 110. The corresponding engagement formation 128 includes a projection that is configured to engage the recess or opening on the body 110. In the illustrated example, the recess or opening is positioned on a first side 110a of the body 110 and spaced "rearward" of the outlet 102 (i.e. towards the collecting volume of the appliance or upstream of the outlet 102). It should be appreciated that the first engagement formation could be a projection that is received by the corresponding engagement formation on the connection member. In other words, one of the first and second engagement formations includes a receiving portion and the other of the first and second engagement formations includes a projection.

In some embodiments, the first engagement formation 112 includes a channel 112a that leads to the recess or opening. The channel 112a provides an additional receiving portion that receives the connection member 122 (in this example, the elongate member 126 of the connection member 122), when the corresponding engagement formation 128 is received in the first engagement formation 112. Thus, when the connection member 122 is connected to the body 110, an external surface of the body 110 is in line with an external surface of the connection member 122 to provide a smooth or continuous external surface (i.e. a profile of the body 110 and connection member 122 connected together (when the closure arrangement 120 is in its closed position) is substantially continuous / generally smooth). In other words, a side of the body 110 to which the closure arrangement 120 connects to has a smooth / continuous outline. This is advantageous because it reduces the possibility that the connection member 122 will catch on external items and become inadvertently detached from the body 110 (this is especially true when a user is using the appliance 10 and not aware that the detachment has happened because they are not using the valve to empty the appliance 10).

According to the invention,
the connection between the closure arrangement 120 and the body 110 permits rotation of the closure arrangement 120 while it remains attached. In other words, the connection member 122 can be rotated while the engagement formation on the body 110 and the corresponding engagement formation are engaged / connected. It should be appreciated that the closure arrangement 120 is not necessarily removable in order to be connected to the body with a rotatable connection. The important functionality is that the joint between the closure arrangement 120 (i.e. the connection member 122) and the body 110 is such that rotational movement (about a single location) of the closure arrangement 120 is permitted.

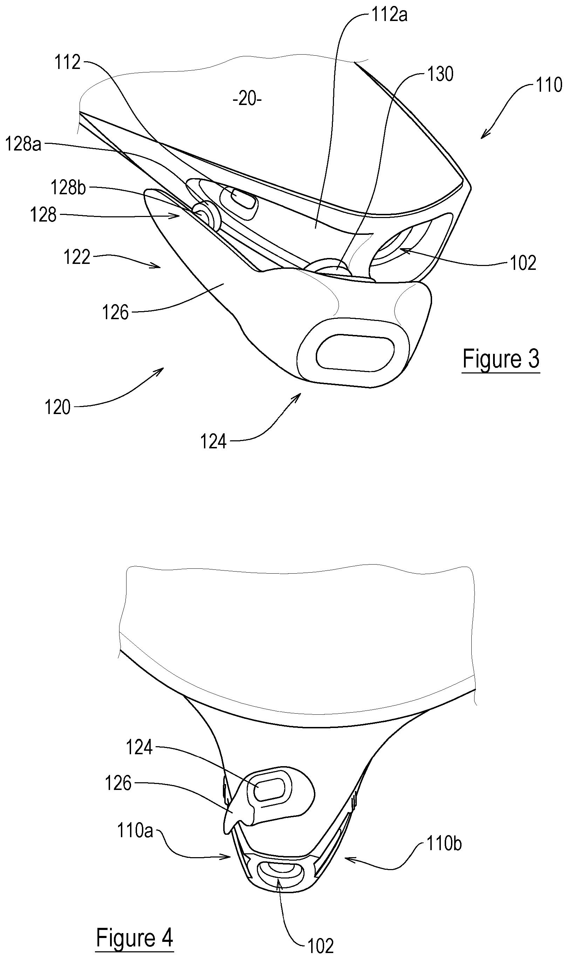

According to the invention
(see particularly figure 3 for a clear view), the corresponding engagement formation 128 of the connection member 122 includes a projection with a domed portion 128a and a narrowed section 128b. The first engagement formation 112 includes a recess wherein the opening at the surface is narrowed with respect to the width of the recess behind. In other words, the opening forms a pinch point. In the described example, at least one of the domed portion 128a and the material of the first engagement formation 112 is formed of a resiliently deformable material. Thus, when the domed portion 128a is inserted through the opening and into the recess, the opening or the domed portion deforms to permit the domed portion 128a through the opening, and then returns to its initial form / shape. Having returned to their initial forms, the material forming the opening resists movement of the domed portion 128a outwards (i.e. away from the body 110) but allows the narrowed portion to rotate (about a generally horizontal axis that extends transverse to the flow of waste through the valve 100) while maintaining the domed portion 128a within the recess.

Therefore, the first engagement formation 112 resists removal of the corresponding formation 128 but allows the closure body to rotate with respect to the body 110.

In some embodiments, the closure arrangement 120 is connectable to the body 110 in two different locations. In other words, the connection member 122 may be attached to the body 110 in a choice of at least two locations or positions. These connection options are in addition to the required positioning of the bung portion 130 with respect to the outlet 102 to close the valve 100 (e.g. the bung portion 130 / closure body 124 is always receivable in the outlet 102 in order to move the closure arrangement 120 to its closed position).

In such an example, the body 110 includes a second engagement formation. Thus, the corresponding engagement formation 128 can engage either the first or the second engagement formation. In this example, the second engagement formation has similar or the same form as the first engagement formation 112, so that the corresponding engagement formation 128 of the connection member 122 can engage either the engagement formation as desired by a user.

In some embodiments, the first receiving formation 112 is located on a first side 110a of the body 110 and the second receiving formation is located on a second side 110b. In the present example, the first and second sides are on opposing sides of the body 110. Thus, the connection member 122 may engage either the first side 110a or the second side 110b of the body 110.

In embodiments where the first and second engagement formations both include the channel 112a that extends towards the recess or opening, the other one of the engagement formations 112 that is not being used by the connection member 122 also has a function. The corresponding engagement formation 128 and the connection member 122 are received in the channel and recess, respectively, to form a substantially continuous external surface. On the opposing side 110a or 110b the channel of the "other" of the first and second engagement formations provides a usable notch for the user to open the valve 100 (i.e. it provides a space for the user to grasp the closure body 124 and to pull it outwards from the outlet 102 and move the closure arrangement 120 to its open position).

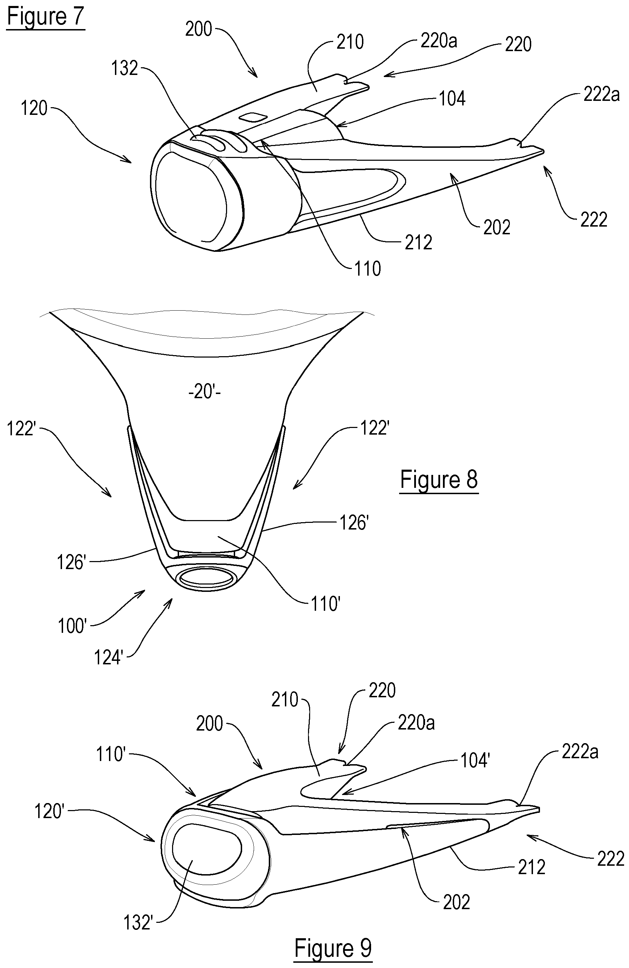

It should be appreciated that the closure arrangement may include two connection members (see for example figure 8 and 9). The embodiment discussed above in which the body 110 includes a first and second engagement formation 112 may allow a closure arrangement to connect on both sides of the body 110 in the same manner as described above in relation to one connection member. Each connection member would have the same formations as the single connection member 122 and connect in analogous manner (this may provide additional connection resilience). Due to the elasticity of the material, the closure arrangement may be rotated outwards from the connection point in the body 110. Both the first and second engagement formations 112 share the same axis of rotation.

In some embodiments, the closure body 124 includes a gripping portion 132 to aid a user moving the closure arrangement 120 to its open position and / or closed position. In the illustrated example (e.g. see figure 7), the gripping portion 132 includes a raised surface which provides a surface that is easier to grip than the smooth surface of the remainder of the closure body 124.

In some embodiments, at least the connection member 122 (and more specifically the elongate portion 126) is formed from an elastic / resiliently deformable material, which allows the connection between the closure arrangement and the body to be flexible and resist permanent deformation.

In some embodiments, the valve 100 (i.e. the body 110 and the closure arrangement 120) is formed from a single material (preferably a soft / pliable or flexible material - e.g. TPE - to provide a comfortable wearing experience for the user). The TPE chosen for the closure arrangement 120 may be a harder shore TPE than the TPE for the body 110.

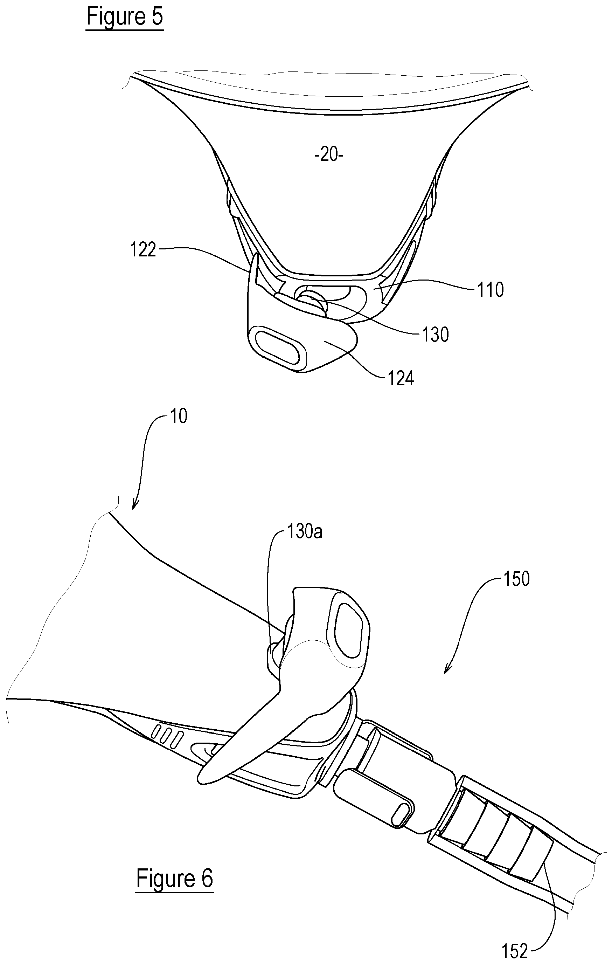

In use, the valve 100 is opened by moving the closure arrangement 120 to its open position. Essentially, this must involve unblocking the outlet 102. Thus, the closure body 124 is moved out of contact with the outlet 102. In addition, the closure arrangement 120 may be detached from the body 110 of the valve 100 (this is shown in detail in figure 3). In some embodiments, the ability to rotate the closure arrangement 120 may also be provided, as discussed above. The ability to leave the closure arrangement 120 attached to the body 110 of the valve 100 while opening the outlet 102 is shown in more detail in figures 4 and 5. Figure 5 shows when the closure body 124 has initially been removed from the outlet 102 - the connection member 122 can be seen flexing while maintaining the connection with the body 110 so that the closure body 124 can be moved away from the outlet 102. Figure 4 shows the closure arrangement 120 rotated up and away from the outlet 102.

If the user is attaching an accessory 150 to their appliance 10 (see, for example, figure 6), the closure arrangement 120 may be moved out of the way and remains connected to the body 110 of the valve 100. Alternatively, the closure arrangement 120 may be removed completely and stored elsewhere while the accessory 150 is in use. Figure 6 illustrates the attachment of a so called "night drainage adaptor" which allows the user to connect a separate tube 152 to their appliance 10 and drain waste into a second collecting vessel to extend the period of time between emptying the appliance 10 (this is particularly useful at night when someone will want to sleep for an extended period of time). Being able to remove the closure arrangement 120 may improve comfort, as the addition material of the valve 100 can be placed elsewhere (not close to the body 110).

A second embodiment of a valve is illustrated in figures 8 to 11. Unless explicitly stated, optional features that relate to the valve illustrated in figures 2 to 7 are equally useable on the valve in figures 8 to 11. Where features of the valve or appliance are the same or similar to those already discussed above they are provided with the same reference number with a prime symbol (i.e. 10 will become 10').

The valve 100' includes the body 110', the inlet 104', the outlet 102' and the closure arrangement 120' as already discussed. Again, the closure arrangement 120' is moveable between a closed position and an open position. Where, in the closed position, the closure arrangement 120' blocks the outlet 102', such that liquid cannot flow through the outlet 102'. And, in the open position, the outlet 102' is open, such that liquid is permitted to flow out of the outlet 102'.

The closure arrangement 120' is connectable / connected to the body 110' in two locations (see particularly figures 8 and 10). In other words, the closure arrangement 120' is connected either permanently or removably in two places on the body 110'. In the illustrated examples, the connections members 122' are permanently attached to the body 110'.

In some embodiments, the closure arrangement 120' includes first and second connection members 122'. Each of the first and second connection members 122' connects to the body 110'. In the illustrated example, the closure arrangement 120' includes a closure body 124' and first and second connection members 122'.

In some embodiments, each connection member 122' includes an elongate portion 126' that extends away from the closure body 124'. In some embodiments (for example, figures 8 onwards), the closure body 124' is generally central and between the two connection members 122'. In other words, the first connection member 122' connects to a first side of the closure body 124' and the second connection member 122' connects to a second side of the closure body 124'. The connection members 122' are on opposing sides of the closure body 124'.

In some embodiments, the first connection member 122' connects to a first side 110a' of the body 110' and the second connection member 122' connects to a second side 110b' of the body 110'. Thus, the connections members 122' are on opposing sides of the body 110' of the valve 100'. In the present example, the connection members 122' forms the external "sides" of the valve 100' - in that the first connection member 122' extends from the first side of the closure body 124' to the first side 110a' of the body 110' and the second connection member 122' extends from the second side of the closure body 124' to the second side 110b' of the body 110'.

Further, in this example, again the closure body 124' supports a bung portion 130' that is configured to be positioned in the outlet 102' when the closure arrangement 120' is in its closed position (i.e. blocking waste from exiting the valve 100'). Thus, the bung portion 130' is held in the outlet 102' by a friction or push fit (as outlined above). In this embodiment, the first and second connection members 122' provide additional biasing force to urge the closure body 124' (and the bung 130') towards the closed position when the closure body is aligned or substantially aligned with the outlet 102'.

Like the single connection member valve version described above, the bung portion 130' includes a flanged part 130a' that engages an internal surface inside the outlet 102'. This may further reinforce the connection between the closure and the body 110' of the valve and reduce the likelihood that the bung potion 130' will detach itself / come out of the outlet 102' accidently.

In some embodiments, the closure body 124' includes a grasping or user aid 132' that is formed to assist the user when grasping and / or moving the closure body 124'. The aid 132' may include a recess / detent for a finger, and / or raised ridges to provide additional grip for the user.

The way in which the valve 100' functions will now be discussed as this is different to the valve 100 described above due to the two connection points between the closure arrangement 120' and the body 110'.

As already mentioned, when the valve 100' is closed (i.e. the closure arrangement 120' is in its closed position), which is illustrated on figures 8 and 9, the closure body 124' is generally aligned with the outlet 102' (which allows the bung portion 130' to be inserted into the outlet 102' to close the valve 100'). In some embodiments, the first and second connection members 122' are formed of a resiliently deformable or biased material. This means that in order to remove the closure body 124' bung portion 130' from the closed position (i.e. move them outwards and away from the body 110'), the connection members 122' are stretched from their original form. Thus, the connection members 122' act to resist removal of the closure body 124' from the closed position. In some embodiments, the connection members 122' apply an additional force on the closure body 124' when in the closed position - thus, the connection members 122' "pull" the closure body 124' into closer contact with the body 110' (i.e. the connections members 122' are deflected from their natural shape / size / configuration and apply a biasing force towards the body 110').

When the closure arrangement 120' is in its open position, it is held or positioned adjacent the body 110'. In some embodiments (see, for example, figures 10 and 11), the closure arrangement 120' is held in a position that contacts the body 110' when it is in its open position. In other words, the closure arrangement 120' is held against the body 110' - the closure body 124' contacts and is pulled against an outside surface (i.e. a front or back surface) of the body 110'. The closure body 124' is located above the bung portion 130' and they are generally aligned / parallel to the body 110' / outlet 102' through the body 110'. The form of the appliance 10 is discussed in more detail below. However, in terms of the position of the closure body 124' when it is open is essentially in front or behind the body 110' and tail arrangement.

To move the closure body 124' from alignment with the outlet 102' (i.e. closed), the closure body 124' can be pulled away from the body 110' and / or transverse force is used to roll the closure body 124' towards the front or back of the body 110'. This movement is against the pull of the connection members 122' on each side of the closure body 124'. A result of this movement means that the closure body 124' can be urged away from the outlet 102'.

In some embodiments, the closure body 124' is moved to an offset position, out of alignment with the outlet 102'. The first and second connection members 122' are deflected and hold the closure body 124' against / in contact with an external surface of the body 110'. As above, because the connection members 122' are made of a resiliently deformable material, they are deflected from their natural configuration and thus apply a biasing force to the closure body 124' towards the side of the body 110'.

When a user wishes to close the valve 100', the closure body 124' must be actively moved from its open position towards the outlet 102' (i.e. a force must be applied to the closure arrangement 120' when it is in the open position to return it to the closed position). This is advantageous because the closure arrangement 120' is held stably in a well-defined / predetermined position away from the outlet in the open position (and against the body 110' of the valve). Previous designs have often relied on a closure being allowed to fly in free space when in the open position, whereas this closure body is held in a position where it will not interfere with further use of the valve 100'.

If the user is attaching an accessory 150' to their appliance 10' (see, for example, figure 11), the closure arrangement 120' may be moved out of the way and is held against the side of the body 110'. Figure 11 illustrates the attachment of a night drainage adaptor which allows the user to connect a separate tube 152' to their appliance 10' and drain waste into a second collecting vessel to extend the period of time between emptying the appliance 10.

Having a predetermined position in which the closure body 124' is held when in the open position improves comfort for the user because it will not hang away from their appliance 10' in a potentially uncomfortable position.

Additional features of the valve will now be described that have an impact on how the valve 100, 100' is attached to the appliance 10, 10'. Figures 7 and 9 illustrate valves that have the features described above already and are illustrated alone and not attached to any appliance 10, 10'. Where features of the valve are discussed, only the reference number without a prime has been used for clarity. However, it should be appreciated that the valve references with a prime symbol are equally applicable.

The body 110 includes a first attachment portion 200 and a second attachment portion 202. Each attachment portion 200, 202 extends away from the inlet 104. In other words, the valve 100 includes a pair of attachment portions 200, 202 which are elongate and project away from the body 110 of the valve 100. In a top down or bottom up view of the valve 100, the attachment portions 200, 202 extend away from the body 110, so that the overall valve shape is generally U or V shaped (i.e. each attachment portion 200, 202 forms a limb).

The outlet 102 of the valve 100 lies downstream of the inlet 104 in a first direction. Each attachment portion 200, 202 extends away from the inlet 104 in a second direction that is opposite to the first direction (i.e., upstream of the inlet 104). In this example, the second direction is generally towards a main collecting volume of the appliance 10, when the valve 100 is connected to the appliance 10.

The first and second attachment portions 200, 202 each provide one or more attachment surfaces for connection to the appliance 10. In the illustrated examples, the attachment portions 200, 202 provide first and second attachment surfaces 210, 212. The first attachment surface 210 lies substantially in a first plane and the second attachment surface 212 lies substantially in a second plane. The first plane and the second plane are inclined with respect to each other. In the illustrated example, the path between the inlet 104 and the outlet 102 extends through the body 110 between the first and second planes (discussed in more detail below).

In some embodiments, the first attachment surface 210 is formed from an external surface of the body 110. The second attachment surface 212 is formed from another external surface of the body 110. In the illustrated example, the external surfaces forming the first and second attachment surfaces are on opposing sides of the valve 100. In other words, the first attachment surface 210 forms an upper surface of the valve 100 and the second attachment surface 212 forms a lower surface of the valve 100.

In this example, the inlet 104 and outlet 102 and the flow path extending between them extends through a generally central portion of the body 110. Thus, the generally central portion of the body 110 extends between the first and second attachment surfaces 210, 212. In other words, (upper and lower) external surfaces of the generally central portion of the body 110 and the first and second attachment portions 200, 202 form the first and second attachment surfaces 210, 212.

In some embodiments, the first and or the second attachment portions 200, 202 taper (i.e. get progressively narrower) as they extend away from the central portion of the body 110 of the valve 100. In other words, the first and second attachment portions 200, 202 each form a general wedge shape when viewed side on. Thus, the first and second planes in which the first and second attachment surfaces 210, 212 generally extend intersect to form a point where the valve ends. In other words, the first and second attachment surfaces 210, 212 meet at an end of first attachment portion 200 and form a first tip 220 (i.e. at one of the ends of the U / V shape). Likewise, at the second attachment portion 202, the first and second attachment surfaces 210, 212 meet to form a second tip 222 (on the other end of the U / V shape).

In some embodiments, one or both of the first and second tips 220, 222 includes a notched portion 220a, 222a. This is advantageous because it aids the manufacturing process and makes it easier to achieve a seal between outlet portion 20, 20' material and the attachment surfaces 210, 212

The valve 100 is attached to the outlet portion 20. In the present example, the first attachment surface 210 is connected to the first outlet wall 22 and the second attachment surface 212 is connected to the second outlet wall 24. Preferably, the outlet portion 20 is heat-welded to the first and second attachment portions 200, 202. In other words, the attachment surfaces 210, 212 are welded to a film layer of the appliance 10 which provides a secure and reliable seal between the components.

Thus, the tail arrangement includes the outlet portion 20 and the valve 100. Internally, the passage for waste (prior to entering the flow path of the valve 100) is formed from a combination of the first and second outlet walls 22, 24 and the first and second attachment portions 200, 202 of the valve 100. At a top part of the outlet portion 20 (i.e. nearest the main first and second walls 12, 14), the first and second outlet walls 22, 24 are attached together. Then, as the valve begins (further away from the collecting volume), the first and second tips 220, 222 are included into the "wall" (i.e. the first and second outlet walls are no longer attached to each other and are attached to the first and second attachment surfaces 210, 212 instead). As more of the valve 100 is incorporated (i.e. closer to the inlet 104 and outlet 102), the attachment portions 200, 202 of the body 110 become gradually wider. Thus, the sides of the tail arrangement get gradually wider on the approach to the inlet 104 / outlet 102. This configuration provides the smooth tapering of the surface of the appliance 10 as previously described.

This arrangement is different to existing valves in which a first part of the outlet flow path from the main collecting volume is completely formed from the first and second outlet walls and a second part of the flow path is formed by the valve. In the present arrangement, there is a transition between the outlet portion 20 and the valve 100. In other words, the waste flow path out of the appliance 10 is formed initially (i.e. in a first part) by the first and second walls 12, 14 / first and second outlet walls 22, 24, then (in a second part) by a combination of the outlet walls 22, 24 and the first and second attachment portions 200, 202 of the valve 100 and then finally (a third part) the waste flow through a path formed entirely by the valve 100.

When used in this specification and claims, the terms "comprises" and "comprising" and variations thereof mean that the specified features, steps or integers are included. The terms are not to be interpreted to exclude the presence of other features, steps or components.

## Claims

1. A valve (100) for a urostomy appliance including:
a body (110) for connection to the urostomy appliance, wherein the body includes a first engagement formation (112)
an inlet (104) and an outlet (102), connected by a flow path, and
a closure arrangement (120) which is moveable between a closed position, in which the closure arrangement (120) blocks the outlet (102), such that liquid cannot flow through the outlet (102), and an open position in which the outlet (102) is open, such that liquid is permitted to flow out of the outlet (102), wherein the closure arrangement (120) is detachably connectable to the body (110), and **characterised in that** the first engagement formation (112) forms a receiving portion including a recess or opening, the valve further includes a connection member (122), including a corresponding engagement formation (128) that is configured to engage with the first engagement formation (112), the corresponding engagement formation (128) includes a projection with a domed portion (128a) and a narrowed section (128b), such that the connection between the closure arrangement (120) and the body (110) is rotatable whilst resisting removal of the closure arrangement (120) from the body (110).

2. A valve (100) according to claim 1 wherein the closure arrangement (120) is detachably connectable to the body (110) in two different locations.

3. A valve (100) according to claim 1 or 2 wherein the body (110) provides a second receiving formation configured to receive a portion of the connection member (122).

4. A valve (100) according to claim 4 wherein the first receiving formation is located on a first side of the body (110) and the second receiving formation is located on a second side.

5. A valve (100) according to claim 5 wherein the first and second sides are on opposing sides of the body (110).

6. A valve (100) according to any of claims 3 to 5 wherein the second receiving formation includes a recess.

7. A valve (100) according to claim 6 wherein the first and / or second receiving formation further includes a channel leading to the recess.

8. A valve (100) according to any of claims 1 to 7 wherein the closure arrangement (120) includes:
a closure body (110) and the connection member (122) includes an elongate portion that extends from away from the closure body (110).

9. A valve (100) according to claim 7 and 8 wherein the connection member (122) includes a projection that extends from the elongate portion to engage the recess.

10. A valve (100) according to claim 9 or 10 wherein the closure body (110) includes a gripping portion to aid a user moving the closure arrangement (120) to its open position and / or closed position

11. A valve (100) according to claims 8 to 10 wherein the elongate portion is formed from an elastic or resiliently deformable material, so that the connection between the closure arrangement (120) and the body (110) is flexible.

12. A valve (100) according to any of the preceding claims wherein the closure arrangement (120) includes a bung portion which is positioned in the outlet (102) when the closure arrangement (120) is in its closed position and preferably, the bung portion is held in the outlet (102) by a friction / push fit.

13. A valve according to claim 12 wherein the bung portion includes a flanged part that engages an internal surface inside the outlet.

14. A urostomy or ileostomy appliance including:
an adhesive member for attaching to a user,
a collecting volume defined by a first and second wall connected about their peripheries, and
a valve (100) according to any of the preceding claims.

## Patentansprüche

1. Ventil (100) für eine Urostomievorrichtung, das Folgendes aufweist:
einen Körper (110) zur Verbindung mit einer Urostomievorrichtung, wobei der Körper ein erstes Eingriffsgebilde (112) aufweist,
einen Einlass (104) und einen Auslass (102), die durch einen Durchflussweg verbunden sind, und
eine Verschlussanordnung (120), die zwischen einer geschlossenen Stellung, in der die Verschlussanordnung (120) den Auslass (102) blockiert, so dass keine Flüssigkeit durch den Auslass (102) fließen kann, und einer offenen Stellung, in der der Auslass (102) offen ist, so dass Flüssigkeit zum Auslass (102) hinausfließen kann, bewegbar ist, wobei die Verschlussanordnung (120) abnehmbar mit dem Körper (110) verbindbar ist, und **dadurch gekennzeichnet, dass** das erste Eingriffsgebilde (112) einen Aufnahmeteil mit einer Aussparung oder Öffnung bildet, wobei das Ventil ferner Folgendes aufweist:
ein Verbindungselement (122) mit einem entsprechenden Eingriffsgebilde (128), das konfiguriert ist, um mit dem ersten Eingriffsgebilde (112) in Eingriff zu sein, wobei das entsprechende Eingriffsgebilde (128) einen Vorsprung mit einem gewölbten Teil (128a) und einem verschmälerten Abschnitt (128b) aufweist, so dass die Verbindung zwischen der Verschlussanordnung (120) und dem Körper (110) drehbar ist, während sie der Abtrennung der Verschlussanordnung (120) vom Körper (110) widersteht.

2. Ventil (100) nach Anspruch 1, wobei die Verschlussanordnung (120) an zwei verschiedenen Stellen abnehmbar mit dem Körper (110) verbindbar ist.

3. Ventil (100) nach Anspruch 1 oder 2, wobei der Körper (110) ein zweites Aufnahmegebilde bereitstellt, das zum Aufnehmen eines Teils des Verbindungselements (122) konfiguriert ist.

4. Ventil (100) nach Anspruch 4, wobei das erste Aufnahmegebilde sich auf einer ersten Seite des Körpers (110) befindet und das zweite Aufnahmegebilde sich auf einer zweiten Seite befindet.

5. Ventil (100) nach Anspruch 5, wobei die erste und die zweite Seite auf entgegengesetzten Seiten des Körpers (110) liegen.

6. Ventil (100) nach einem der Ansprüche 3 bis 5, wobei das zweite Aufnahmegebilde eine Aussparung aufweist.

7. Ventil (100) nach Anspruch 6, wobei das erste und/oder zweite Aufnahmegebilde ferner eine zu einer Aussparung führende Nut aufweist.

8. Ventil (100) nach einem der Ansprüche 1 bis 7, wobei die Verschlussanordnung (120) Folgendes aufweist:
einen Verschlusskörper (110), und das Verbindungselement (122) einen länglichen Teil aufweist, der sich vom Verschlusskörper (110) weg erstreckt.

9. Ventil (100) nach Anspruch 7 und 8, wobei das Verbindungselement (122) einen Vorsprung aufweist, der sich vom länglichen Teil erstreckt, um mit der Aussparung in Eingriff zu kommen.

10. Ventil (100) nach Anspruch 9 oder 10, wobei der Verschlusskörper (110) einen Griffteil aufweist, um einem Benutzer beim Bewegen der Verschlussanordnung (120) in seine offene Stellung und/oder geschlossene Stellung behilflich zu sein.

11. Ventil (100) nach einem der Ansprüche 8 bis 10, wobei der längliche Teil aus einem elastischen oder elastisch verformbaren Material hergestellt ist, so dass die Verbindung zwischen der Verschlussanordnung (120) und dem Körper (110) flexibel ist.

12. Ventil (100) nach einem der vorhergehenden Ansprüche, wobei die Verschlussanordnung (120) einen Stöpselteil aufweist, der in dem Auslass (102) positioniert ist, wenn die Verschlussanordnung (120) in ihrer geschlossenen Stellung ist, und, vorzugsweise, der Stopfenteil durch Reibschluss/Schiebepassung im Auslass (102) gehalten wird.

13. Ventil nach Anspruch 12, wobei der Stöpselteil ein Flanschteil aufweist, das an einer Innenfläche im Inneren des Auslasses in Anlage ist.

14. Urostomie- oder Ileostomievorrichtung, die Folgendes aufweist:
ein Klebeelement zum Anbringen an einem Benutzer,
ein Sammelvolumen, das von einer ersten und einer zweiten Wand definiert wird, die an ihrem Umfang jeweils miteinander verbunden sind, und
ein Ventil (100) nach einem der vorhergehenden Ansprüche.

## Revendications

1. Valve (100) pour appareil d'urostomie, comprenant :
un corps (110) destiné à être relié à l'appareil d'urostomie, le corps comprenant une première formation d'engagement (112),
une entrée (104) et une sortie (102), reliées par une voie d'écoulement, et
un agencement de fermeture (120) qui est mobile entre une position fermée dans laquelle l'agencement de fermeture (120) bloque la sortie (102), de telle sorte que le liquide ne puisse pas s'écouler par la sortie (102), et une position ouverte dans laquelle la sortie (102) est ouverte, de telle sorte que le liquide puisse s'écouler hors de la sortie (102), l'agencement de fermeture (120) pouvant être relié amovible au corps (110), et
la valve étant **caractérisée en ce que** la première formation d'engagement (112) forme une partie de réception comprenant un évidement ou une ouverture, la valve comprend en outre un élément de liaison (122), comprenant une formation d'engagement correspondante (128) qui est configurée pour s'engager avec la première formation d'engagement (112), la formation d'engagement correspondante (128) comprend une saillie avec une partie bombée (128a) et une section rétrécie (128b), de telle sorte que la liaison entre l'agencement de fermeture (120) et le corps (110) soit rotative tout en résistant au retrait de l'agencement de fermeture (120) du corps (110).

2. Valve (100) selon la revendication 1, l'agencement de fermeture (120) pouvant être relié amovible au corps (110) à deux emplacements différents.

3. Valve (100) selon la revendication 1 ou 2, le corps (110) produisant une seconde formation de réception configurée pour recevoir une partie de l'élément de liaison (122).

4. Valve (100) selon la revendication 4, la première formation de réception étant située sur un premier côté du corps (110) et la seconde formation de réception étant située sur un second côté.

5. Valve (100) selon la revendication 5, les premier et second côtés étant situés sur des côtés opposés du corps (110).

6. Valve (100) selon l'une quelconque des revendications 3 à 5, la seconde formation de réception comprenant un évidement.

7. Valve (100) selon la revendication 6, la première et/ou la seconde formation de réception comprenant en outre un canal menant à l'évidement.

8. Valve (100) selon l'une quelconque des revendications 1 à 7, l'agencement de fermeture (120) comprenant :
un corps de fermeture (110), et l'élément de liaison (122) comprenant une partie allongée qui s'étend depuis l'opposé du corps de fermeture (110).

9. Valve (100) selon les revendications 7 et 8, l'élément de liaison (122) comprenant une saillie qui s'étend depuis la partie allongée pour s'engager dans l'évidement.

10. Valve (100) selon la revendication 9 ou 10, le corps de fermeture (110) comprenant une partie de préhension pour aider un utilisateur à déplacer l'agencement de fermeture (120) vers sa position ouverte et/ou sa position fermée.

11. Valve (100) selon les revendications 8 à 10, la partie allongée étant formée à partir d'un matériau élastique ou déformable par résilience, de telle sorte que la liaison entre l'agencement de fermeture (120) et le corps (110) soit flexible.

12. Valve (100) selon l'une quelconque des revendications précédentes, l'agencement de fermeture (120) comprenant une partie bouchon qui est positionnée dans la sortie (102) lorsque l'agencement de fermeture (120) est dans sa position fermée et, de préférence, la partie bouchon étant maintenue dans la sortie (102) par un ajustement par friction/pression.

13. Valve selon la revendication 12, la partie bouchon comprenant une pièce bridée qui s'engage sur une surface interne à l'intérieur de la sortie.

14. Appareil d'urostomie ou d'iléostomie comprenant :
un élément adhésif destiné à être fixé à un utilisateur,
un volume de collecte défini par une première et une seconde paroi reliées au niveau de leur périphérie, et
une valve (100) selon l'une quelconque des revendications précédentes.
